# FASCICULE DE BREVET EUROPEEN

(11) **EP 0 927 176 B1**
(45) Date de publication et mention de la délivrance du brevet: **30.05.2001**
(21) Numéro de dépôt: 97933742.5
(22) Date de dépôt: 15.07.1997
(51) Int. Cl.: C07D 319/06

(54) **DERIVES DE 1,3-DIOXANE, PROCEDE DE SYNTHESE ET COMPOSITIONS AROMATIQUES LES CONTENANT**
1,3-DIOXAN DERIVATE, VERFAHREN ZU IHRER HERSTELLUNG UND AROMATISIERENDE ZUSAMMENSETZUNGEN DIE DIESE ENTHALTEN
1,3-DIOXANE DERIVATIVES, SYNTHESIS METHOD THEREFOR AND AROMATIC COMPOSITIONS CONTAINING SAME

(30) Priorité: 16.07.1996 FR 9608850
(43) Date de publication de la demande: 07.07.1999
(73) Titulaire: PERNOD-RICARD, 75008 Paris (FR)
(72) Inventeur: BRUNERIE, Pascal, Marc, F-94440 Santeny (FR)
(74) Mandataire: Warcoin, Jacques
(86) Numéro de dépôt international: FR9701310
(87) Numéro de publication internationale: WO9802429

(56) Documents cités:
- JANUSZ KULESZA ET AL.: "Ausnutzung der n-alpha-Olefine C6-C11 bei der Synthese der Riechstoffe II. Teil" RIECHSTOFFE AROMEN KORPERPFLEGEMITTEL, vol. 19, no. 4, avril 1969, HANNOVER-BEMRODE DE, page 157 XP000647556
- V. V. KUTZNETSOV ET AL.: "Alkylboronic Acid Ester in Reaction of Olefins with Paraform" RUSSIAN JOURNAL OF GENERAL CHEMISTRY, vol. 64, no. 10, octobre 1994, NTS BUREAU US, pages 1555-1556, XP002027956
- ADAM SOKOLOWSKI ET AL.: "Acetals and Ethers. 11. Solubility of Alkyl- Substituted 1,3-Dioxolanes and 1,3-Dioxanes in Water" THE JOURNAL OF PHYSICAL CHEMISTRY, vol. 88, no. 4, 16 février 1984, pages 807-809, XP000646189
- IZUMI YAJIMA ET AL.: "Volatile Flavor Components of Kogyoku Apples" AGRICULTURAL AND BIOLOGICAL CHEMISTRY, vol. 48, no. 4, avril 1984, TOKYO JP, pages 849-855, XP000647555

## Description

La présente invention concerne les composés de structure 1,3-dioxane provenant de la pomme et des produits dérivés de la pomme, ainsi que le procédé de synthèse desdits composés de structure 1,3-dioxane par acétalisation des diols-1,3 correspondants.

La présente invention repose sur la mise en évidence, dans les pommes, de la présence de composés de structure 1,3-dioxane présentant des propriétés organoleptiques intéressantes et également de leurs précurseurs diol-1,3 présents en grande quantité. Ceci a permis d'envisager une synthèse de ces composés de structure 1,3-dioxane à partir de composés diols-1,3 naturels ou obtenus par voie de synthèse en utilisant certains des aldéhydes connus comme étant présents dans la fraction volatile de la pomme.

Les réactions d'acétalisation entre un composé possédant une fonction alcool et un composé possédant une fonction aldéhyde ou cétone se produisent facilement en conditions acides et ont été largement étudiées (Vollhardt, 1988 ; Ogata et al., 1970 ; Schmitz et al., 1967 ; Sandler et al., 1972).

Chaque étape de la réaction d'acétalisation étant équilibrée, une modification des conditions de réaction peut favoriser la formation d'acétals, par exemple en éliminant l'eau formée au cours de la réaction ou par utilisation d'un excès d'alcool (Meskens et al., 1981). De plus, la réaction effectuée à partir de diol-1,2 ou -1,3 conduit à la formation d'acétals cycliques dont la stabilité déplace l'équilibre en leur faveur. En outre, certains de ces acétals cycliques possèdent des propriétés organoleptiques intéressantes et sont ainsi utilisés dans l'industrie cosmétique, notamment dans la préparation de parfums (Naarden International, 1974 ; Henkel, 1976 Kulesza, J., et al., Riechstoffe, Aromen, Körperpflegemittel, 19(4), (1969), 157.

Les études consacrées aux composés volatils de la pomme et des produits qui en sont dérivés, effectuées par la demanderesse, ont permis de mettre en évidence la présence de nouveaux composés aromatisants aux propriétés sensorielles intéressantes. C'est pourquoi, la présente invention concerne plus particulièrement les composés, de formule qénérale I : 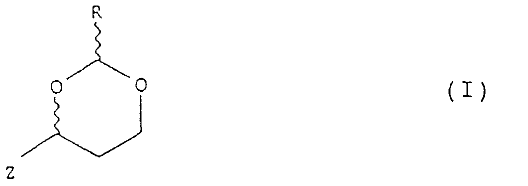 dans laquelle
Z est le groupement pentyle ou pent-2-ényle (Z) ou (E) et R est un groupement hydrocarboné qui peut être aliphatique ou non aliphatique, notamment un radical alkyle, substitué ou non, ou aromatique, substitué ou non, par exemple phényle.

Les radicaux alkyles comportent, de préférence de 1 à 6 atomes de carbone. Parmi les substituants, il faut citer les radicaux alkyles en C₁ à C₆, ou des substituants tels que halo, OH ou NH₂ par exemple.

La présente invention concerne notamment les composés de formule générale I caractérisés en ce qu'ils proviennent de la pomme et de ses produits dérivés, c'est-à-dire en particulier les composés de formule générale I dans lesquels le radical R est en C₁ à C₅.

La formule I couvre aussi bien les différents stéréoisomères lorsqu'ils existent que les mélanges desdits stéréoisomères, notamment les mélanges racémiques.

L'invention concerne également les composés sous formes cis ou trans, de préférence sous forme cis.

Bien que ces composés de formule générale I puissent être obtenus par extraction à partir de sources naturelles, notamment de la pomme ou de ses produits dérivés tels que le cidre, ces composés peuvent également être obtenus par synthèse chimique.

Ces composés peuvent, en effet, être obtenus par acétalisation :
- du diol-1,3 correspondant de formule générale II : 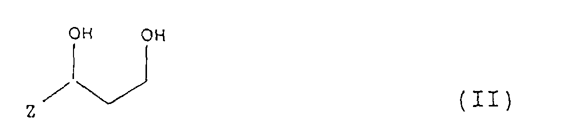 dans laquelle Z est défini ci-dessus, notamment les diols naturellement présents en grande quantité dans certaines variétés de pommes à cidre,
- par un aldéhyde de formule : 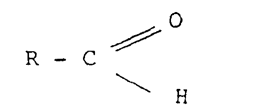 lequel est également présent naturellement dans la pomme, comme l'éthanal, le propanal, le butanal et l'hexanal.

L'acétalisation peut être catalysée dans le procédé selon l'invention par un acide, de préférence en présence d'un agent desséchant.

Comme cela sera développé plus en détail, il est particulièrement intéressant de partir de composés d'origine naturelle, notamment de produits provenant de la pomme, par exemple pour les composés de formule générale II.

En effet, si l'obtention par voie de synthèse de l'octanediol-1,3 (diol-1,3 de formule générale II avec Z = pentyle) ne présente aucune difficulté, celle de son homologue insaturé, l'octène-5-diol-1,3 (diol-1,3 de formule générale II avec Z = pent-2-ényle) est délicate et il est préférable de partir du produit d'extraction de la pomme ou de ses produits dérivés, dans lesquels ces deux composés sont présents.

Les aldéhydes sont, quant à eux bien connus, même s'ils peuvent également être obtenus par extraction à partir de sources naturelles telles que la pomme.

Le procédé de synthèse, selon l'invention, des composés 4-(pent-2-ényl)-1,3-dioxane (1,3-dioxane de formule générale I avec Z = pent-2-ényle) peut donc être mis en oeuvre à partir de l'octène-5-diol-1,3 provenant de la pomme ou des produits dérivés de la pomme, ou, de préférence, à partir d'un mélange des deux diols-1,3,l'octanediol-1,3 et l'octène-5-diol-1,3, provenant tous deux de la pomme ou des produits dérivés de la pomme, le diol-1,3 insaturé étant difficile à isoler du mélange.

Par contre, la synthèse des composés 4-pentyl-1,3-dioxane (1,3-dioxane de formule générale I avec Z = pentyle) peut être effectuée aussi bien à partir de l'octanediol-1,3 isolé de la pomme ou des produits dérivés de la pomme, qu'à partir de l'octanediol-1,3 obtenu par voie de synthèse.

Les diols-1,3, saturés et insaturés, naturels, sont isolés de la pomme ou des produits dérivés de la pomme par toute méthode appropriée, en particulier par extraction du broyat de pomme ou du produit dérivé par un solvant organique, par exemple le dichlorométhane.

Les composés de structure 1,3-dioxane selon l'invention, de formule générale I, sont obtenus de préférence à partir d'un diol-1,3 ou d'un mélange de diols-1,3, mis en présence d'une quantité stoechiométrique d'aldéhyde ou d'un mélange de plusieurs aldéhydes, de préférence l'éthanal, le propanal, le butanal, l'hexanal, le tout dans un solvant polaire, notamment un éther aliphatique, par exemple l'éther éthylique. Une quantité catalytique d'acide, en particulier l'acide para-toluène sulfonique, peut être ajoutée au mélange réactionnel ainsi qu'un agent desséchant destiné à l'élimination de l'eau formée au cours de la réaction. On choisira, par exemple, comme agent desséchant le sulfate de sodium anhydre. Le milieu réactionnel est maintenu à une température comprise entre 10 et 40°C, sous agitation, pendant 6 à 24 heures, de préférence 12 heures à 25°C. Le milieu est ensuite filtré et lavé à l'eau, le solvant éliminé par distillation et les composés de structure 1,3-dioxane de formule générale I sont ensuite purifiés par toute méthode appropriée, notamment par distillation ou par séparation sur colonne de silice.

Dans le cas où l'on fait réagir un mélange de diols-1,3 et/ou un mélange d'aldéhydes au cours du procédé de synthèse des composés de structure 1,3-dioxane selon l'invention, on peut avoir recours à la chromatographie en phase gazeuse (CG) afin de séparer chaque composé du mélange de 1,3-dioxanes obtenu à l'issue dudit procédé.

Chaque composé 1,3-dioxane de formule générale I, lorsqu'il est obtenu à partir des diols-1,3 de structure générale I ou II de synthèse, possède deux carbones asymétriques en positions 2 ét 4 : 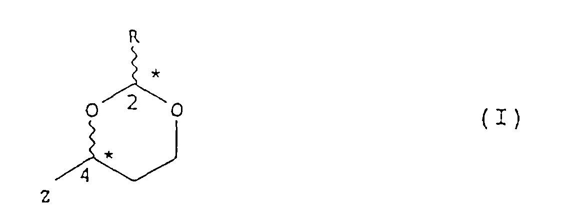 chacun de ces composés se présente donc sous la forme d'un mélange de deux diastéréoisomères ou encore d'un mélange de quatre énantiomères. Les composés de structure 1,3-dioxane naturellement présents dans la pomme et les produits dérivés de la pomme, en particulier les composés naturels:
- 2-méthyl-4-pentyl-1,3-dioxane,
- 2-éthyl-4-pentyl-1,3-dioxane,
- 2-propyl-4-pentyl-1,3-dioxane, et
- 2,4-dipentyl-1,3-dioxane,
analysés par chromatographie en phase gazeuse sur colonne chirale, apparaissent sous la forme majoritaire des énantiomères 4R mais, si l'on part de produits de synthèse, il est possible d'obtenir d'autres énantiomères.

On peut analyser les diastéréoisomères des composés de formule générale I, obtenu par le procédé de synthèse selon l'invention par chromatographie en phase gazeuse sur colonne normale.

L'analyse par chromatographie en phase gazeuse sur colonne chirale de chaque diastéréoisomère permet ensuite la séparation de quatre énantiomères. Cette ultime séparation permet l'analyse des quatre isomères de configuration du composé de formule générale I obtenu par voie de synthèse selon le procédé de l'invention.

L'ensemble des composés de formule générale I obtenus par le procédé de synthèse selon l'invention possèdent des propriétés organoleptiques intéressantes, que l'on peut déterminer par analyse sensorielle.

Les 1,3-dioxanes selon la présente invention peuvent être utilisés dans des compositions, notamment en parfumerie ou dans l'industrie agroalimentaire, afin d'aromatiser lesdites compositions.

En particulier, ces composés possèdent des propriétés nouvelles intéressantes et peuvent être utilisés, notamment, pour introduire des notes vertes, fraîches et fruitées.

Les exemples suivants illustreront la présente invention sans en limiter la portée.

### EXEMPLE 1 : Préparation des composés 4-pentyl-1,3-dioxane

L'octanediol-1,3 (0,5g, 4mM) purifié est mis en présence d'une quantité stoechiométrique d'aldéhyde (éthanal, propanal, butanal ou hexanal) dans 5m1 d'éther comme solvant. Une quantité catalytique d'acide para-toluène sulfonique est ajoutée ainsi que du sulfate de sodium anhydre (pour éliminer l'eau formée au cours de la réaction). Le milieu réactionnel est laissé à température ambiante sous agitation pendant 12 heures.

Le milieu est ensuite filtré et lavé à l'eau, l'éther éliminé par distillation et les composés 4-pentyl-1,3-dioxane sont ensuite purifiés par distillation ou par chromatographie sur colonne de silice.

Les rendements obtenus sont les suivants :

| Aldéhyde reactant | 1,3-Dioxane obtenu | Rendement (%) |
|---|---|---|
| Ethanal | 1:R=méthyl:2-méthyl-4-pentyl-1,3-dioxane | 91 |
| Propanal | 2:R=éthyl:2-éthyl-4-pentyl-1,3-dioxane | 77 |
| Butanal | 3:R=propyl:2-propyl-4-pentyl-1,3-dioxane | 41 |
| Hexanal | 4:R=pentyl:2,4-dipentyl-1,3-dioxane | 75 |

### Caractérisatiques de distillation

| Acétal formé | Pression (mBar) | Point d'ébullition (°C) | Pureté (GC) (%) |
|---|---|---|---|
| R = méthyl | 20 | 78 | 99,4 |
| R = éthyl | 15 | 83 | 99,5 |
| R = propyl | 15 | 98,5 | 99,3 |
| R = pentyl | 18 | 130 | 98,7 |

### EXEMPLE 2 : Préparation des composés 4-(pent-2-ényle)-1,3-dioxane

On suit le même mode opératoire que celui décrit dans l'exemple 1 en partant d'un mélange d'octanediol-1,3 et de cis-octène-5-diol-1,3 (4mM) provenant tous deux de la pomme.

On obtient un mélange du dérivé 1,3-dioxane saturé (composé 1, 2, 3 ou 4) et de son homologue insaturé (composé 5, 6, 7 ou 8 respectivement). 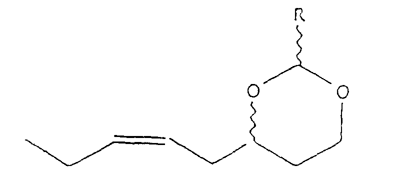
5: R = -méthyl : 2-méthyl-4-[(Z)-pent-2-ényl]-1,3-dioxane,
6: R = -éthyl : 2-éthyl-4-[(Z)-pent-2-ényl]-1,3-dioxane,
7: R = -propyl : 2-propyl-4-[(Z)-pent-2-ényl]-1,3-dioxane,
8: R = -pentyl : 2-pentyl-4-[(Z)-pent-2-ényl]-1,3-dioxane.

Du fait des très faibles quantités de cis-octène-5-diol-1,3 présentes naturellement dans la pomme et de l'utilisation du mélange octanediol-1,3/cis-octène-5-diol-1,3, les rendements de formation des dérivés de 4-(pent-2-ényl)-1,3-dioxane n'ont pu être calculés. Par contre, chacun des composés 5 à 8 a été analysé par chromatographie en phase gazeuse couplée à la spectrométrie de masse et caratérisé par son spectre de masse.

## Revendications

1. Composés de structure 1,3-dioxane de formule qénérale I : 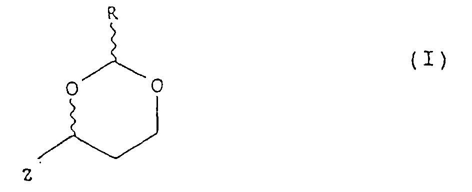 dans laquelle
Z est le groupement pentyle ou pent-2-ényle (Z) ou (E) et
R est un groupement hydrocarboné.

2. Composés selon la revendication 1, caractérisés en ce qu'ils sont obtenus à partir de la pomme ou de ses produits dérivés.

3. Composés selon la revendication 1, caractérisés en ce que R est un radical aliphatique en C₁ à C₆ ou un radical aromatique substitué ou non substitué.

4. Composés selon la revendication 1, caractérisés en ce que R est un radical alkyle en C₁ à C₆.

5. Composé selon l'une des revendications 1 à 4, caractérisé en ce qu'il est sous forme cis.

6. Procédé de synthèse de composés de structure 1,3-dioxane selon l'une des revendications 1 à 5, caractérisé en ce qu'on acétalise le diol-1,3 correspondant de formule générale II : 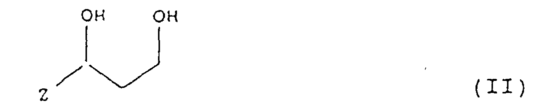 dans laquelle Z est tel que défini précédemment, par un aldéhyde de formule : 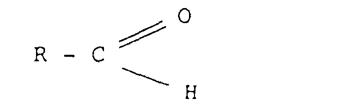 dans laquelle R a la signification donnée précédemment.

7. Procédé selon la revendication 6,
caractérisé en ce que le diol-1,3 de formule générale II est d'origine naturelle ou de synthèse.

8. Procédé selon l'une des revendications 6 et 7, caractérisé en ce que l'aldéhyde est d'origine naturelle ou de synthèse.

9. Procédé selon l'une des revendications 6 à 8, caractérisé en ce que l'aldéhyde est un aldéhyde aliphatique en C₂-C₇.

10. Procédé selon l'une quelconque des revendications 6 à 9, caractérisé en ce que l'acétalisation est catalysée par un acide, en particulier l'acide para-toluène sulfonique.

11. Procédé selon l'une quelconque des revendications 6 à 10, caractérisé en ce que l'acétalisation est réalisée dans un solvant polaire, en particulier dans l'éther éthylique.

12. Procédé selon l'une quelconque des revendications 6 à 11, caractérisé en ce que l'acétalisation est réalisée en présence d'un agent desséchant, en particulier le sulfate de sodium anhydre.

13. Procédé selon l'une quelconque des revendications 6 à 12, caractérisé en ce que le composé de structure 1,3-dioxane est obtenu sous la forme d'un mélange de diastéréoisomères.

14. Procédé selon la revendication 13, caractérisé en ce que chaque énantiomère de chacun des diastéréoisomères est séparé par chromatographie en phase gazeuse sur colonne chirale.

15. Compositions contenant un composé de structure 1,3-dioxane de formule générale I selon l'une des revendications 1 à 5 ou obtenu par le procédé décrit selon les revendications 6 à 14.

## Claims

1. Compounds having a 1,3-dioxane structure of general formula I: 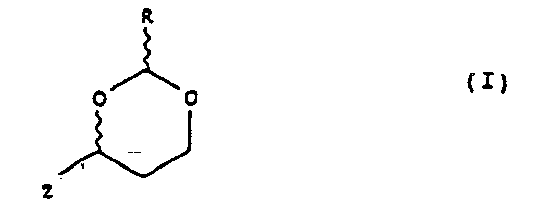 in which
Z is the pentyl or (Z) or (E) pent-2-enyl group and
R is a hydrocarbon group.

2. Compounds according to Claim 1, characterized in that they are obtained from apple or products derived therefrom.

3. Compounds according to Claim 1, characterized in that R is a C₁ to C₆ aliphatic radical or an aromatic radical which is substituted or otherwise.

4. Compounds according to Claim 1, characterized in that R is a C₁ to C₆ alkyl radical.

5. Compound according to one of Claims 1 to 4, characterized in that it is in cis form.

6. Method of synthesis of compounds having a 1,3-dioxane structure according to one of Claims 1 to 5, characterized in that the corresponding 1,3-diol of general formula II: 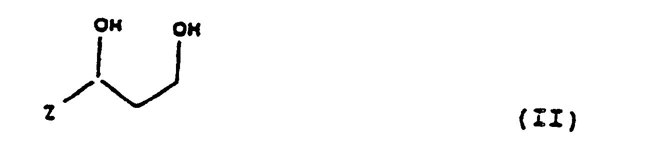 in which Z is as defined above,
is acetalized with an aldehyde of formula: 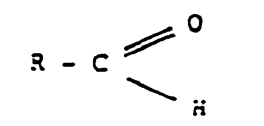 in which R has the meaning given above.

7. Method according to Claim 6, characterized in that the 1,3-diol of general formula II is of natural or synthetic origin.

8. Method according to either of Claims 6 and 7, characterized in that the aldehyde is of natural or synthetic origin.

9. Method according to one of Claims 6 to 8, characterized in that the aldehyde is a C₂-C₇ aliphatic aldehyde.

10. Method according to any one of Claims 6 to 9, characterized in that the acetalization is catalyzed by an acid, in particular para-toluenesulfonic acid.

11. Method according to any one of Claims 6 to 10, characterized in that the acetalization is carried out in a polar solvent, in particular in ethyl ether.

12. Method according to any one of Claims 6 to 11, characterized in that the acetalization is carried out in the presence of a drying agent, in particular anhydrous sodium sulfate.

13. Method according to any one of Claims 6 to 12, characterized in that the compound having a 1,3-dioxane structure is obtained in the form of a mixture of diastereoisomers.

14. Method according to Claim 13, characterized in that each enantiomer of each of the diastereoisomers is separated by gas chromatography on a chiral column.

15. Compositions containing a compound having a 1,3-dioxane structure of general formula I according to one of Claims 1 to 5 or obtained by the method described according to Claims 6 to 14.

## Patentansprüche

1. Verbindungen mit 1,3-Dioxanstruktur der allgemeinen Formel I: 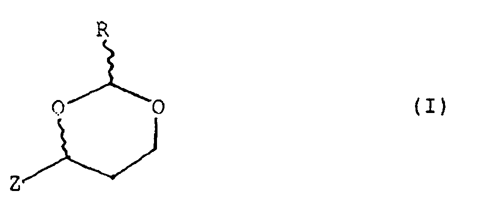 worin
Z eine Pentyl- oder Pent-2-enylgruppe (Z) oder (E) und R eine Kohlenwasserstoffgruppe sind.

2. Verbindungen gemäß Anspruch 1, dadurch gekennzeichnet, dass sie ausgehend vom Apfel oder seinen abgeleiteten Produkten erhalten werden.

3. Verbindungen gemäß Anspruch 1, dadurch gekennzeichnet, dass R ein aliphatisches Radikal mit C₁ bis C₆ oder ein substituiertes oder nicht substituiertes aromatisches Radikal ist.

4. Verbindungen gemäß Anspruch 1, dadurch gekennzeichnet, dass R ein Alkylradikal mit C₁ bis C₆ ist.

5. Verbindung gemäß einem der Ansprüche 1 bis 4, dadurch gekennzeichnet, dass sie in cis-Form vorliegt.

6. Verfahren zur Herstellung von Verbindungen mit 1,3-Dioxanstruktur gemäß einem der Ansprüche 1 bis 5, dadurch gekennzeichnet, dass man ein 1,3-Diol entsprechend der allgemeinen Formel II: 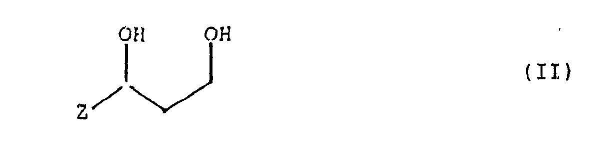 worin Z die zuvor beschriebene Bedeutung hat,
mit einem Aldehyd der Formel: 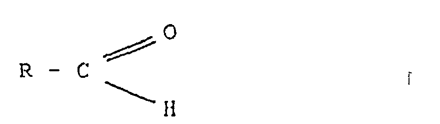 worin R die zuvor beschriebene Bedeutung hat,
acetalisiert.

7. Verfahren gemäß Anspruch 6, dadurch gekennzeichnet, dass das 1,3-Diol der allgemeinen Formol II natürlichen oder synthetischen Ursprungs ist.

8. Verfahren gemäß einem der Ansprüche 6 und 7, dadurch gekennzeichnet, dass das Aldehyd natürlichen oder synthetischen Ursprungs ist.

9. Verfahren gemäß einem der Ansprüche 6 bis 8, dadurch gekennzeichnet, dass das Aldehyd ein aliphatisches Aldehyd mit C₁ - C₇ ist.

10. Vorfahren gemäß wenigstens einem der Ansprüche 6 bis 9, dadurch gekennzeichnet, dass die Acetalisierung von einer Säure, insbesondere para-Toluolsulfonsäure, katalysiert wird.

11. Verfahren gemäß wenigstens einem der Ansprüche 6 bis 10, dadurch gekennzeichnet, dass die Acetalisierung in einem polaren Lösungsmittel, insbesondere in Ethylether, durchgeführt wird.

12. Vorfahren gemäß wenigstens einem der Ansprüche 6 bis 11, dadurch gekennzeichnet, dass die Acetalisierung in Gegenwart eines Trockenmittels, insbesondere wasserfreiem Natriumsulfat:, durchgeführt wird.

13. Verfahren gemäß wenigstens einem der Ansprüche 6 bis 12, dadurch gekennzeichnet, dass die Verbindung mit 1,3-Dioxanstruktur in Form einer Mischung von Diastereoisomeren erhalten wird.

14. Verfahren gemäß Anspruch 13, dadurch gekennzeichnet, dass jedes Enantiomere der jeweiligen Diastereoisomeren durch Gasphasenchromatographie auf einer chiralen Säule abgetrennt wird.

15. Zusammensetzungen, welche eine Verbindung mit 1,3-Dioxanstruktur der allgemeinen Formel I gemäß einem der Ansprüche 1 bis 5 oder erhalten durch das in den Ansprüchen 6 bis 14 beschriebene Verfahren enthalten.
